(19) Europäisches Patentamt
European Patent Office
Office européen des brevets

(11) EP 3 591 064 A1

(12) EUROPEAN PATENT APPLICATION
published in accordance with Art. 153(4) EPC

(43) Date of publication:
**08.01.2020 Bulletin 2020/02**

(21) Application number: **18760684.3**

(22) Date of filing: **28.02.2018**

(51) Int Cl.:
***C12P 21/02*** (2006.01) ***C12P 21/00*** (2006.01)

(86) International application number:
**PCT/JP2018/007424**

(87) International publication number:
**WO 2018/159655 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **01.03.2017 JP 2017038403**

(71) Applicant: **Sanyo Chemical Industries, Ltd.**
**Kyoto 605-0995 (JP)**

(72) Inventors:
- **SOMAMOTO, Satoshi**
  **Kyoto-shi**
  **Kyoto 605-0995 (JP)**
- **OOHORA, Satoe**
  **Kyoto-shi**
  **Kyoto 605-0995 (JP)**
- **NAKANISHI, Mutsumi**
  **Kyoto-shi**
  **Kyoto 605-0995 (JP)**

(74) Representative: **Mewburn Ellis LLP**
**City Tower**
**40 Basinghall Street**
**London EC2V 5DE (GB)**

# (54) METHOD FOR PRODUCING USEFUL MATERIAL

(57) The present invention provides a method of producing a useful substance, including secreting and producing a useful substance in broth by microorganisms contained in the broth, wherein the microorganisms include microorganisms of at least one genus selected from the group consisting of *Ogataea, Saccharomyces, Kluyveromyces, Hansenula, Pichia, Yarrowia,* and *Candida,* and the broth contains at least one surfactant (A) selected from the group consisting of an anionic surfactant (A1), a cationic surfactant (A2), and an amphoteric surfactant (A3).

EP 3 591 064 A1

## Description

### TECHNICAL FIELD

The present invention relates to a method of producing a useful substance.

### BACKGROUND ART

Yeast has been widely used to produce useful substances such as amino acids and proteins. Particularly in recent years, a technique has been known in which proteins are efficiently produced by yeast transformed by introducing a medically and/or industrially useful protein gene thereinto.

Use of Escherichia coli as a bacterium for production of useful substances is limited to production of proteins without sugar moieties because a glycan synthetase is absent in Escherichia coli. In contrast, use of yeast for production of useful substances can produce proteins with sugar moieties attached because proteins expressed in the yeast are secreted out of the bacterium after the proteins are modified by post-translational modification such as glycosylation by a glycan synthetase.

Yet, the protein production capacity of the protein production method that uses yeast is unfortunately lower than that of the method that uses Escherichia coli. In order to solve the problem, a technique has been reported which increases the amount of protein secretion by the use of a PMR1 gene-disrupted strain of *S.cerevisiae* or *Yarrowia lipolytica* to reduce the thickness of the cell walls of yeast (Non-Patent Literature 1). Yet, unfortunately, this method also has a low the protein production capacity.

### CITATION LIST

- Non-Patent Literature

Non-Patent Literature 1: J. Bacteriol. 1998, vol. 180, no. 24, pp. 6736-6742

### SUMMARY OF INVENTION

- Technical Problem

The present invention aims to provide a method of producing a useful substance capable of efficiently secreting and producing a useful substance by microorganisms.

- Solution to Problem

As a result of extensive studies to solve the above problems, the present inventors arrived at the present invention.

Specifically, the present invention provides a method of producing a useful substance, including secreting and producing a useful substance in broth by microorganisms contained in the broth, wherein the microorganisms include microorganisms of at least one genus selected from the group consisting of *Ogataea, Saccharomyces, Kluyveromyces, Hansenula, Pichia, Yarrowia,* and *Candida,* and the broth contains at least one surfactant (A) selected from the group consisting of an anionic surfactant (A1), a cationic surfactant (A2), and an amphoteric surfactant (A3).

- Advantageous Effects of Invention

The method of producing a useful substance of the present invention can efficiently secrete and produce a useful substance by microorganisms.

### DESCRIPTION OF EMBODIMENTS

The present invention provides a method of producing a useful substance including secreting and producing a useful substance in broth by microorganisms contained in the broth, wherein the microorganisms include microorganisms of at least one genus selected from the group consisting of *Ogataea, Saccharomyces, Kluyveromyces, Hansenula, Pichia, Yarrowia,* and *Candida,* and the broth contains at least one surfactant (A) selected from the group consisting of an anionic surfactant (A1), a cationic surfactant (A2), and an amphoteric surfactant (A3).

Any broth can be used in the production method of the present invention as long as it is a cell culture medium

commonly used in the relevant technical field. Either a natural medium or synthetic medium containing a carbon source, a nitrogen source, and other essential nutrients may be used.

Examples of the carbon source include carbohydrates such as glucose, fructose, sucrose, and starch; organic acids such as acetic acid and propionic acid; and alcohols such as ethanol and propanol.

Examples of the nitrogen source include ammonia, ammonium salts of inorganic acids or organic acids (e.g., ammonium chloride, ammonium sulfate, ammonium acetate, and ammonium phosphate), other nitrogen-containing compounds, peptone, meat extract, and corn steep liquor.

Examples of the other essential nutrients include inorganic salts. Examples of the inorganic salts include monopotassium phosphate, dipotassium phosphate, magnesium phosphate, magnesium sulfate, sodium chloride, ferrous sulfate, manganese sulfate, copper sulfate, and calcium carbonate.

In terms of production of the useful substance, the microorganisms in the present invention include microorganisms of at least one genus selected from the group consisting of *Ogataea, Saccharomyces, Kluyveromyces, Hansenula, Pichia, Yarrowia,* and *Candida,* most preferably at least one genus selected from the group consisting of *Saccharomyces, Pichia,* and *Candida.*

In the present invention, the broth contains at least one surfactant (A) selected from the group consisting of an anionic surfactant (A1), a cationic surfactant (A2), and an amphoteric surfactant (A3).

The anionic surfactant (A1), the cationic surfactant (A2), and the amphoteric surfactant (A3) are different from nonionic surfactants, and are considered to be ionic surfactants.

Specifically, the surfactant (A) in the present invention is an ionic surfactant.

In terms of secretion efficiency of the useful substance, the pKa of the ionizable group in these surfactants (A) in water is preferably 1 to 5 and/or 8 to 14, more preferably 1 to 4 and/or 9 to 14. The pKa of the ionizable group in water is a value determined at 25°C.

Specific examples of the ionizable group having a pKa of 1 to 5 include acidic groups and their salts, such as carboxy (-COOH), sulfate ($-OSO_3H$), sulfo ($-SO_3H$), sulfino ($-SO_2H$), sulfeno (-SOH), and phosphate ($-OP(=O)(-OH)_2$) groups.

Specific examples of the ionizable group having a pKa of 8 to 14 include quaternary ammonio groups, primary to tertiary amino groups, and their salts.

When the surfactant (A) contains multiple ionizable groups, in terms of secretion efficiency of the useful substance, preferably, one of these ionizable groups in the surfactant (A) has a pKA in the above range, and more preferably, all of these ionizable groups have a pKA in the above range.

Examples of the anionic surfactant (A1) include an ether carboxylic acid (A11) and its salts, a sulfate ester (A12) and its salts, an ether sulfate ester (A13) and its salts, a sulfonate (A14), a sulfosuccinate (A15), a phosphate ester (A16) and its salts, an ether phosphate ester (A17) and its salts, a fatty acid salt (A18), an acylated amino acid salt, and a naturally occurring carboxylic acid and its salts (e.g., chenodeoxycholic acid, cholic acid, and deoxycholic acid).

Examples of the ether carboxylic acid (A11) and its salts include ether carboxylic acids having a hydrocarbon group (C8-C24) and their salts.

The ether is preferably an alkylene oxide adduct, more preferably at least one adduct of ethylene oxide or propylene oxide, particularly preferably an ethylene oxide adduct, in terms of secretion efficiency of the useful substance and cytotoxicity.

The degree of polymerization of the alkylene oxide is preferably 1 to 10, in terms of secretion efficiency of the useful substance and cytotoxicity.

Specific examples of the ether carboxylic acid (A11) and its salts include polyoxyethylene lauryl ether acetic acid, sodium polyoxyethylene lauryl ether acetate, sodium polyoxyethylene tridecyl ether acetate, sodium polyoxyethylene octyl ether acetate, and sodium lauryl glycol acetate.

Examples of the sulfate ester (A12) and its salts include sulfate esters having a hydrocarbon group (C8-C24) and their salts. Specific examples of the sulfate ester (A12) and its salts include sodium lauryl sulfate and a lauryl sulfate triethanolamine salt.

Examples of the ether sulfate ester (A13) and its salts include ether sulfate esters having a hydrocarbon group (C8-C24) and their salts.

The ether is preferably an alkylene oxide adduct, more preferably at least one adduct of ethylene oxide or propylene oxide, particularly preferably an ethylene oxide adduct, in terms of secretion efficiency of the useful substance and cytotoxicity.

The degree of polymerization of the alkylene oxide is preferably 1 to 10, in terms of secretion efficiency of the useful substance and cytotoxicity.

Specific examples of the ether sulfate ester (A13) and its salts include sodium polyoxyethylene lauryl ether sulfate and polyoxyethylene lauryl ether sulfate triethanolamine salt.

Examples of the sulfonate (A14) include sodium dodecyl diphenyl ether disulfonate, sodium dodecyl benzene sulfonate, and sodium naphthalenesulfonate.

Examples of the sulfosuccinate (A15) include disodium polyoxyethylene lauryl sulfosuccinate, disodium lauryl sulfosuccinate, and disodium polyoxyethylene lauroyl ethanolamide sulfosuccinate.

Examples of the phosphate ester (A16) and its salts include disodium octyl phosphate and disodium lauryl phosphate.

Examples of the ether phosphate ester (A17) and its salts include disodium polyoxyethylene octyl ether phosphate and disodium polyoxyethylene lauryl ether phosphate.

Examples of the fatty acid salt (A18) include sodium octanoate, sodium laurate, and sodium stearate.

The anionic surfactant (A1) is preferably a compound represented by the following formula (1).

[Chem. 1]

$$R^1\text{—}(OA)_s\text{—}Q \qquad (1)$$

In the formula (1), $R^1$ represents a C1-C30 monovalent hydrocarbon group; (OA) represents an oxyalkylene group (e.g., oxyethylene, oxypropylene, or oxybutylene); s is an integer of 1 or more; and Q represents a sulfonic acid (salt) group, a carboxylic acid (salt) group, or a phosphoric acid (salt) group.

The "sulfonic acid (salt)" means sulfonic acid and/or sulfonate; the "carboxylic acid (salt)" means carboxylic acid and/or carboxylate; and the "phosphoric acid (salt)" means phosphoric acid and/or phosphate. Examples of salts include alkali metal salts (e.g., sodium salts and potassium salts), alkaline earth metal salts (e.g., calcium salts and magnesium salts), and onium cation salts (e.g., ammonium cations, quaternary ammonium cations, tertiary sulfonium cations, quaternary phosphonium cations, and tertiary oxonium cations).

In the formula (1), in terms of secretion efficiency of the useful substance and cytotoxicity, $R^1$ is preferably a C8-C22 monovalent hydrocarbon group, more preferably a C10-C18 monovalent hydrocarbon group.

In terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

In terms of secretion efficiency of the useful substance and cytotoxicity, the oxyalkylene group is preferably an oxyethylene group.

In terms of secretion efficiency of the useful substance and cytotoxicity, s is preferably an integer of 1 to 10, more preferably an integer of 1 to 5.

Examples of the compound represented by the formula (1) include sodium polyoxyethylene (average 2.5 mol adduct) lauryl ether sulfate, and sodium polyoxyethylene (average 3 mol adduct) lauryl ether acetate.

Examples of the cationic surfactant (A2) include an amine salt cationic surfactant (A21) and a quaternary ammonium salt cationic surfactant (A22).

Examples of the amine salt cationic surfactant (A21) include primary to tertiary amines neutralized by an inorganic acid (e.g., hydrochloric acid, nitric acid, sulfuric acid, or hydroiodic acid) or by an organic acid (e.g., acetic acid, formic acid, oxalic acid, lactic acid, gluconic acid, adipic acid, or alkyl phosphate). Examples of the primary amine salt cationic surfactant include inorganic acid salts or organic acid salts of higher aliphatic amines (higher amines such as laurylamine, stearylamine, cetylamine, hardened beef tallow amine, and rosin amine); and salts of higher fatty acids (e.g., stearic acid and oleic acid) of lower amines. Examples of the secondary amine salt cationic surfactant include inorganic acid salts or organic acid salts of ethylene oxide adducts of aliphatic amines. Examples of the tertiary amine salt cationic surfactant include inorganic acid salts or organic acid salts of aliphatic amines (e.g., triethylamine, ethyldimethylamine, and N,N,N',N'-tetramethylethylenediamine), ethylene oxide (2 mol or more) adducts of aliphatic amines, alicyclic amines (e.g., N-methylpyrrolidine, N-methylpiperidine, N-methylhexamethyleneimine, N-methylmorpholine, and 1,8-diazabicyclo(5,4,0)-7-undecene), and nitrogen-containing heterocyclic aromatic amines (e.g., 4-dimethylaminopyridine, N-methylimidazole, and 4,4'-dipyridyl); and inorganic acid salts or organic acid salts of tertiary amines such as triethanolamine monostearate and stearamideethyldiethylmethylethanolamine.

Examples of the quaternary ammonium salt cationic surfactant (A22) include one obtainable by a reaction of a tertiary amine with a quaternarizing agent (e.g., an alkylating agent such as methyl chloride, methyl bromide, ethyl chloride, benzyl chloride, or dimethylsulfuric acid; or ethylene oxide). Examples include lauryl trimethyl ammonium chloride, didecyl dimethyl ammonium chloride, dioctyl dimethyl ammonium bromide, stearyl trimethyl ammonium bromide, lauryl dimethyl benzyl ammonium chloride (benzalkonium chloride), cetylpyridinium chloride, polyoxyethylene trimethyl ammonium chloride, and stearamideethyldiethylmethyl ammonium methosulfate.

The quaternary ammonium salt cationic surfactant (A22) is preferably a compound represented by the following formula (2), in terms of secretion efficiency of the useful substance and cytotoxicity.

[Chem. 2]

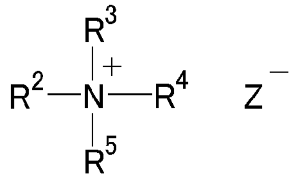

(2)

In the formula (2), $R^2$, $R^3$, $R^4$, and $R^5$ each represent a C1-C30 monovalent hydrocarbon group, and $Z^-$ represents a counter anion.

In the formula (2), in terms of secretion efficiency of the useful substance and cytotoxicity, preferably at least one of $R^2$, $R^3$, $R^4$, or $R^5$ is a C8-C22 monovalent hydrocarbon group, and more preferably, at least one of $R^2$, $R^3$, $R^4$, or $R^5$ is a C10-C18 monovalent hydrocarbon group.

In $R^2$, $R^3$, $R^4$ and $R^5$, in terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

$Z^-$ is preferably a halogen ion, more preferably a chloride ion, in terms of secretion efficiency of the useful substance and cytotoxicity.

In the formula (2), the hydrocarbon group may have at least one substituent selected from the group consisting of hydroxyl, ether, carbonyl, and ester groups at any position of the hydrocarbon group.

Examples of the amphoteric surfactant (A3) include a carboxylate amphoteric surfactant (A31), a sulfate amphoteric surfactant (A32), a sulfonate amphoteric surfactant (A33), and a phosphate amphoteric surfactant (A34).

Examples of the carboxylate amphoteric surfactant (A31) include an amino acid amphoteric surfactant (A311), a betaine amphoteric surfactant (A312), and an imidazoline amphoteric surfactant (A313).

Examples of the amino acid amphoteric surfactant (A311) include a compound which is an amphoteric surfactant having an amino group and a carboxyl group in the molecule and which is represented by the following formula (3).

[Chem. 3]

$$\left[ R^6\text{—NH—}(CH_2)_n\text{—}COO^- \right]_m \quad M \qquad (3)$$

In the formula (3), $R^6$ is a C1-C30 monovalent hydrocarbon group; n is an integer of 1 or more; m is an integer of 1 or more; and M is a proton or a monovalent or divalent cation such as an alkali metal, an alkaline earth metal, ammonium (including a cation derived from amine, alkanolamine, or the like), or quaternary ammonium.

In $R^6$, in terms of secretion efficiency of the useful substance and cytotoxicity, the carbon number of the hydrocarbon group is preferably 8 to 22, more preferably 10 to 18.

In $R^6$, in terms of secretion efficiency of the useful substance and cytotoxicity, the hydrocarbon group is preferably an aliphatic hydrocarbon group, more preferably a linear or branched aliphatic hydrocarbon group having 0 to 3 unsaturated bonds, particularly preferably an alkyl group or an alkenyl group having 1 to 3 unsaturated bonds.

Specific examples of the amino acid amphoteric surfactant (A311) include alkylamino propionate amphoteric surfactants (e.g., sodium dodecyl-β-aminopropionate, sodium cocaminopropionate, sodium stearylaminopropionate, and sodium laurylaminopropionate); alkylaminoacetate amphoteric surfactants (e.g., sodium laurylaminoacetate); and sodium N-lauroyl-N'-carboxymethyl-N'-hydroxyethylethylenediamine.

The betaine amphoteric surfactant (A312) is an amphoteric surfactant having a quaternary ammonium salt cationic site and a carboxylic acid anionic site in the molecule. Examples of the betaine amphoteric surfactant (A312) include a compound represented by the following formula (4).

[Chem. 4]

$$R^7\text{—}N^+(CH_3)_2\text{—}CH_2COO^- \qquad (4)$$

In the formula (4), $R^7$ is a C1-C30 monovalent hydrocarbon group. In $R^7$, in terms of secretion efficiency of the useful substance and cytotoxicity, the carbon number of the hydrocarbon group is preferably 8 to 22, more preferably

10 to 18.

In $R^7$, in terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

Specific examples of the betaine amphoteric surfactant (A312) include alkyl dimethyl betaines (e.g., betaine stearyl dimethylaminoacetate and betaine lauryl dimethylaminoacetate), amide betaines (e.g., coconut oil fatty acid amide propyl betaines (e.g., betaine coconut oil fatty acid amide propyldimethylaminoacetate) and lauric acid amide propyl betaine), alkyldihydroxyalkyl betaines (e.g., lauryl dihydroxyethyl betaine), and betaine hardened coconut oil fatty acid amide propyldimethylaminoacetate.

Examples of the imidazoline amphoteric surfactant (A313) include 2-alkyl-N-carboxymethyl-N-hydroxyethyl imidazolinium betaine.

Examples of other amphoteric surfactants include glycine amphoteric surfactants such as sodium lauroyl glycine, sodium lauryl diaminoethyl glycine, lauryl diaminoethyl glycine hydrochloride, and dioctyl diaminoethyl glycine hydrochloride; sulfobetaine amphoteric surfactants such as pentadecylsulfotaurine; cholamidopropyl dimethylammonio propanesulfonate (CHAPS) and cholamidopropyldimethylammonio-2-hydroxy propanesulfonate (CHAPSO); and alkylamine oxide amphoteric surfactants such as lauryldimethylamine oxide.

In terms of secretion efficiency of the useful substance and cytotoxicity, the amphoteric surfactant (A3) is preferably any one of compounds represented by the following formulas (5) to (7).

[Chem. 5]

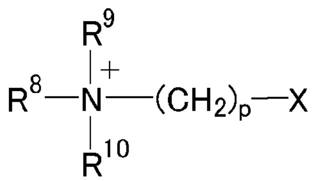

(5)

[Chem. 6]

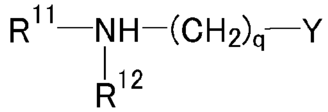

(6)

[Chem. 7]

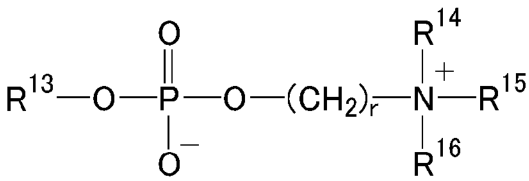

(7)

In the formula (5), $R^8$, $R^9$, and $R^{10}$ each represent a C1-C30 monovalent hydrocarbon group; p is an integer of 1 or more; and X represents a sulfonate anion, carboxylate anion, or phosphonate anion.

In the formula (6), $R^{11}$ and $R^{12}$ each represent a C1-C30 monovalent hydrocarbon group; q is an integer of 1 or more; and Y represents a sulfonic acid (salt) group, carboxylic acid (salt) group, or phosphoric acid (salt) group.

In formula (7), $R^{13}$, $R^{14}$, $R^{15}$, and $R^{16}$ each represent a C1-C30 monovalent hydrocarbon group; and r is an integer of 1 or more.

The "sulfonic acid (salt)" means sulfonic acid and/or sulfonate; the "carboxylic acid (salt)" means carboxylic acid and/or carboxylate, and the "phosphoric acid (salt)" means phosphoric acid and/or phosphate. Examples of salts include alkali metal salts (e.g., sodium salts and potassium salts), alkaline earth metal salts (e.g., calcium salts and magnesium salts), and onium cation salts (e.g., ammonium cations, quaternary ammonium cations, tertiary sulfonium cations, quaternary phosphonium cations, and tertiary oxonium cations).

In the formulas (5) to (7), the hydrocarbon group may have at least one substituent selected from the group

consisting of hydroxyl, ether, carbonyl, and ester groups at any position of the hydrocarbon group.

In the formula (5), in terms of secretion efficiency of the useful substance and cytotoxicity, preferably, at least one of $R^8$, $R^9$, or $R^{10}$ is a C8-C22 monovalent hydrocarbon group, and more preferably, at least one of $R^8$, $R^9$, or $R^{10}$ is a C10-C18 monovalent hydrocarbon group.

$R^8$, $R^9$, and $R^{10}$, in terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

In terms of secretion efficiency of the useful substance and cytotoxicity, p is preferably an integer of 1 to 10, more preferably an integer of 1 to 5.

In the formula (6), in terms of secretion efficiency of the useful substance and cytotoxicity, preferably, at least one of $R^{11}$ or $R^{12}$ is a C8-C22 monovalent hydrocarbon group, and more preferably, at least one of $R^{11}$ or $R^{12}$ is a C10-C18 monovalent hydrocarbon group.

In $R^{11}$ and $R^{12}$, in terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

In terms of secretion efficiency of the useful substance and cytotoxicity, q is preferably an integer of 1 to 10, more preferably an integer of 1 to 5.

In terms of secretion efficiency of the useful substance and cytotoxicity, the salt is preferably an alkali metal salt, more preferably a sodium salt.

In the formula (7), in terms of secretion efficiency of the useful substance and cytotoxicity, preferably, at least one of $R^{13}$, $R^{14}$, $R^{15}$, or $R^{16}$ is a C8-C20 monovalent hydrocarbon group, and more preferably, at least one of $R^{13}$, $R^{14}$, $R^{15}$, or $R^{16}$ is a C10-C18 monovalent hydrocarbon group.

In $R^{13}$, $R^{14}$, $R^{15}$, and $R^{16}$, in terms of secretion efficiency of the useful substance and cytotoxicity, the hydrocarbon group is preferably an aliphatic hydrocarbon group, more preferably a linear and/or branched aliphatic hydrocarbon group having 0 to 3 unsaturated bonds, particularly preferably an alkyl group or an alkenyl group having 1 to 3 unsaturated bonds.

In terms of secretion efficiency of the useful substance and cytotoxicity, r is preferably an integer of 1 to 10, more preferably an integer of 1 to 5.

Specific examples of the compound represented by the formula (5) include those in which X is a sulfonate anion (e.g., dodecyldimethyl(3-sulfopropyl)ammonium hydroxide, 3-[tetradecyldimethylammonio]propane-1-sulfonate, and arachidyl dimethyl(3-sulfopropyl)ammonium hydroxide); those in which X is a carboxylate anion (e.g., betaine lauryldimethylaminoacetate and betaine stearyl dimethylaminoacetate); and those in which X is a phosphonate anion (dodecyldimethyl(3-phosphopropyl)ammonium hydroxide).

Specific examples of the compound represented by the formula (6) include those in which Y is a sulfonic acid (salt) (e.g., sodium (2-dodecylamino)ethanesulfonate, sodium 2-[(1-oxododecyl)amino]ethanesulfonate, and sodium 2-(N-methyl-N-palmitoylamino)ethanesulfonate); those in which Y is a carboxylic acid (salt) (e.g., sodium 3-(dodecylamino)propanoate and sodium dodecyl-β-aminopropionate); and those in which Y is a phosphoric acid (salt) (e.g., sodium (2-dodecylamino)ethanephosphate).

Specific examples of the compound represented by the formula (7) include miltefosine, dodecylphosphorylcholine, hexadecylphosphorylcholine, and 1,2-dihexadecanoyl-sn-glycero-3-phosphocholine.

The surfactant (A) is preferably a compound represented by any one of the formulas (1), (2), and (5) to (7), in terms of secretion efficiency of the useful substance and cytotoxicity.

Among the compounds represented by the formulas (1), (2), and (5) to (7), the surfactant (A) is more preferably a compound having a C8-C22 aliphatic hydrocarbon group, particularly preferably a compound having a C10-C18 aliphatic hydrocarbon group.

In the present invention, the surfactant (A) may be used as-is, or may be mixed with water, if necessary, to be used as an aqueous dilute solution (in the form of aqueous solution or aqueous dispersion).

The total concentration (wt%) of the surfactant (A) in the aqueous dilute solution may be determined as appropriate according to the types of the target microorganisms and physiologically active substances and the type of extraction method. Yet, in terms of secretion efficiency of the useful substance and handleability, the total concentration is preferably 0.1 to 99 wt%, more preferably 1 to 50 wt%, based on the weight of the aqueous dilute solution.

The amount of the surfactant (A) (wt%) used in the method of producing a useful substance of the present invention may be determined as appropriate according to the types of the target microorganisms and the useful substance to be produced and the type of extraction method. Yet, in terms of cytotoxicity, secretion efficiency of the useful substance, and less possibility of causing denaturation of the protein, the amount is preferably 0.0001 to 20 wt%, more preferably 0.001 to 10 wt%, still more preferably 0.005 to 5 wt%, particularly preferably 0.01 to 2.5 wt%, based on the weight of the broth.

Examples of the useful substance produced by the method of producing a useful substance of the present

invention include proteins (e.g., enzymes, hormone proteins, antibodies, and peptides), polysaccharides, oligosaccharides, and nucleic acids.

Examples of proteins include enzymes such as oxidoreductases (e.g., cholesterol oxidase, glucose oxidase, ascorbic acid oxidase, and peroxidase), hydrolases (e.g., lysozyme, protease, serine protease, amylase, lipase, cellulase, and glucoamylase), isomerases (e.g., glucose isomerase), transferases (e.g., acyltransferase and sulfotransferase), synthetases (fatty acid synthase, phosphate synthase, and citrate synthase), and lyases (e.g., pectin lyase); hormone proteins such as osteogenic factor (BMP), interferon $\alpha$, interferon $\beta$, interleukins 1 to 12, growth hormone, erythropoietin, insulin, granulocyte-colony stimulating factor (G-CSF), tissue plasminogen activator (TPA), natriuretic peptide, factor VIII, somatomedin, glucagon, growth hormone-releasing factor, serum albumin, and calcitonin; antibodies; antigen proteins (e.g., hepatitis B surface antigen); functional proteins such as pronectin (registered trademark), antifreeze peptide, and antimicrobial peptide; fluorescent protein (e.g., GFP); luminescent proteins (e.g., luciferase); and peptides (the amino acid composition is not limited; e.g., oligopeptide, dipeptide, and tripeptide).

Examples of polysaccharides include hyaluronic acid, chondroitin, xanthan, and cellulose.

Examples of oligosaccharides include sucrose, lactose, trehalose, maltose, raffinose, panose, cyclodextrin, galactooligosaccharides, and fructooligosaccharides.

Examples of nucleic acids include inosine monophosphate, adenosine monophosphate, and guanosine monophosphate.

In terms of secretory productivity of the useful substance, proteins are preferred.

In the culturing step, broth to be used in the production of the useful substance is sterilized in an autoclave (preferably, heating at 120°C for 20 minutes), and precultured recombinant microorganisms are cultured in this broth. The temperature for microorganism cultivation is preferably 15°C to 32°C, more preferably 25°C to 30°C.

Preferably, the pH of the medium is preferably adjusted to 4 to 7.

Preferably, the culturing step is performed as follows, for example: the surfactant (A) is added to the broth 6 to 800 hours after the start of culturing while the broth temperature is maintained, and the culturing is continued for 20 to 1000 hours.

The broth during culturing is preferably stirred using a known stirrer (e.g., a stirring blade or a magnetic stirrer).

A known culture device can be used in the culturing step. Examples include test tubes, deep well plates (e.g., products of BM Equipment Co., Ltd.), and microbial culture devices (e.g., products of ABLE Corporation).

When recombinant microorganisms are used as the precultured microorganisms to be used in the culturing step, the recombinant microorganisms are produced by transforming microorganisms with an expression vector and the produced recombinant microorganisms are precultured. Preferably, the preculturing is performed in an agar medium at 15°C to 32°C for 3 to 72 hours.

The purification step is a step of isolating the useful substance (e.g., protein) secreted in the broth. The useful substance can be separated from microorganisms and microbial residues by a known separation method such as precipitation (e.g., centrifugal separation, hollow fiber separation, filtration, or precipitation by solvent) or column chromatography treatment (ion exchange column, gel filtration column, hydrophobic column, affinity column, or ultrafiltration column).

Examples of precipitation by solvent include ethanol precipitation, ammonium sulfate precipitation, and polyethylene glycol precipitation.

When the purification step involves column treatment, examples of filler to be used in the column chromatography include silica, dextran, agarose, cellulose, acrylamide, and vinyl polymer. Commercial products such as Sephadex series, Sephacryl series, Sepharose series (all available from Pharmacia), and Bio-Gel series (available from Bio-Rad) are available.

The microorganisms isolated in the purification step can be further cultured in fresh broth. The useful substance can be produced continuously by repeating purifying the broth or the like in the purification step and culturing.

## EXAMPLES

The present invention is described in further detail with reference to the following examples and comparative examples, but the present invention is not limited thereto.

### <Examples 1 to 11>

Transformation was performed using *Saccharomyces* cerevisiae distributed by Biotechnology Center, National Institute of Technology and Evaluation, whereby luciferase-expressing microorganisms were produced. A vector pYES2 (Thermo Fisher Scientific) was digested with HindIII and XbaI, and a synthetic (synthesized by Thermo Fisher Scientific on commission) $\alpha$ factor-luciferase gene having a HindIII cut site at one end and an XbaI cut site at the other end was inserted into the vector. The microorganisms were transformed by the $\alpha$ factor-luciferase gene-inserted pYES2 according

to the method described in an instruction manual of pYES2. The luciferase-expressing microorganisms were inoculated using a platinum loop into YPD broth (yeast extract 1 wt% (Difco), bacto peptone 2 wt% (Difco), and glucose 2 wt%) and were shake-cultured at 200 rpm at 30°C for 15 hours. Then, the thus-obtained broth was resuspended in 125-ml YPD broth (containing 100 mM potassium hydrogen phosphate buffer; pH: 6) to a final optical density (hereinafter sometimes abbreviated as "OD") of 0.1 OD/ml.

The optical density of the broth was measured under the same conditions as for "Method of measuring the optical density of broth" described later.

Further, the resuspended broth was continued to be shake-cultured at 1100 rpm at 30°C for about 17 hours until OD was 20 to 30. When OD was 20 to 30, the broth was centrifuged (1500 g, 10 min), and resuspended in 125-ml galactose-inducing broth (the YPD broth containing 100 mM potassium phosphate buffer and also containing 2% galactose instead of glucose; pH: 6), followed by culturing at 1100 rpm at 30°C for 2 hours.

Subsequently, the surfactant described in Table 1 was added to the broth in the amount (wt%) described in Table 1, and the broth was cultured at 1100 rpm at 30°C for 8 hours. Subsequently, the broth was sampled for measuring the optical density (optical density at the end of culturing) and for measuring the secretion efficiency. Then, these samples were subjected to centrifugation using a centrifuge to separate bacteria and the supernatant from the broth. Then, the supernatant and the bacteria were collected.

<Comparative Example 1>

The supernatant and bacteria were collected in the same manner as in Example 1, except that pure water was added instead of the surfactant.

<Examples 12 to 22>

Transformation was performed using *Pichia pastoris* distributed by Biotechnology Center, National Institute of Technology and Evaluation, whereby luciferase-expressing microorganisms were produced. A vector pPICZ $\alpha$ (Thermo Fisher Scientific) was digested with XhoI and XbaI, and a synthetic (synthesized by Thermo Fisher Scientific on commission) luciferase gene having an XhoI cut site at one end and an XbaI cut site at the other end was inserted into the vector. The microorganisms were transformed by the luciferase gene-inserted pPICZ $\alpha$ according to the method described in an instruction manual of pPICZ $\alpha$. The luciferase-expressing microorganisms were inoculated using a platinum loop into BMGY broth, and were shake-cultured at 200 rpm at 30°C for 15 hours. Then, the thus-obtained broth was resuspended in 125-ml BMGY broth (yeast extract 1 wt% (Difco), bacto peptone 2 wt% (Difco), yeast nitrogen base w/o amino acid 1.34 wt% (Difco), glucose 2 wt%, and 100 mM potassium hydrogen phosphate; pH: 6.0) to a final OD of 0.1 OD/ml. Further, the resuspended broth was continued to be shake-cultured at 1100 rpm at 30°C for about 17 hours until OD was 20 to 30. When OD was 20 to 30, the broth was centrifuged (1500 g, 10 min), and resuspended in 125-ml BMMY broth, followed by culturing at 1100 rpm at 30°C for 2 hours.

Two hours later, the surfactant described in Table 2 was added to the broth in the amount (wt%) described in Table 2, and the broth was cultured at 1100 rpm at 30°C for 8 hours. Subsequently, the broth was sampled for measuring the optical density (optical density at the end of culturing) and for measuring the secretion efficiency. Then, these samples were subjected to centrifugation using a centrifuge to separate bacteria and the supernatant from the broth. Then, the supernatant and the bacteria were collected.

<Comparative Example 2>

The supernatant and bacteria were collected in the same manner as in Example 12, except that pure water was added instead of the surfactant.

<Examples 23 to 33>

*Candida boidinii* was transformed and acid phosphatase-expressing microorganisms were produced based on a method described in a known document (Regulation and evaluation of five methanol-inducible promoters in the methylotrophic yeast *Candida boidinii,* H. Yurimoto et al., Biochimica et Biophysica Acta, 1493, 2000, pp. 56-63). The acid phosphatase-expressing microorganisms were inoculated using a platinum loop into BMGY broth, and were shake-cultured at 200 rpm at 30°C for 15 hours. Then, the thus-obtained broth was resuspended in 125-ml BMGY broth to a final OD of 0.1 OD/ml. Further, the resuspended broth was continued to be shake-cultured at 1100 rpm at 30°C for about 17 hours until OD was 2 to 5. When OD was 2 to 5, methanol was added to a concentration of 2 v/v%, followed by culturing at 1100 rpm at 30°C for 2 hours.

Two hours later, the surfactant described in Table 3 was added to the broth in the weight described in Table

3, and the broth was cultured at 1100 rpm at 30°C for 8 hours. Eight hours later, the broth was sampled for measuring the optical density (optical density at the end of culturing), and was subjected to centrifugation using a centrifuge to separate bacteria and the supernatant from the broth. Then, the supernatant and the bacteria were collected.

Then, 50 mM acetic acid NaBf (pH 4.0) was added to the bacteria, followed by centrifugation, whereby the bacteria were washed. The washed bacteria were resuspended in 50 mM acetic acid NaBf (pH 4.0), whereby a sample for measuring the acid phosphatase activity on the bacterial surface was obtained.

<Comparative Example 3>

The supernatant and bacteria were collected in the same manner as in Example 23, except that pure water was added instead of the surfactant.

[Table 1]

| | | Example | | | | | | | | | | | Comparative Example |
|---|---|---|---|---|---|---|---|---|---|---|---|---|---|
| | | 1 | 2 | 3 | 4 | 5 | 6 | 7 | 8 | 9 | 10 | 11 | 1 |
| Surfactant | Surfactant (A1-1) | - | - | - | - | 0.03 | - | - | - | - | - | - | - |
| | Surfactant (A1-2) | - | - | - | - | - | 0.03 | - | - | - | - | - | - |
| | Surfactant (A2-1) | - | - | - | - | - | - | - | - | - | - | 0.03 | - |
| | Surfactant (A3-1) | - | - | - | - | - | - | 0.03 | - | - | - | - | - |
| | Surfactant (A3-2) | 0.003 | 0.03 | 0.3 | 3 | - | - | - | - | - | - | - | - |
| | Surfactant (A3-3) | - | - | - | - | - | - | - | 0.03 | - | - | - | - |
| | Surfactant (A3-4) | - | - | - | - | - | - | - | - | 0.03 | - | - | - |
| | Surfactant (A3-5) | - | - | - | - | - | - | - | - | - | 0.03 | - | - |
| Evaluation results | Optical density (OD) of broth at the end of culturing | 23.5 | 21.2 | 18.2 | 10.9 | 21.9 | 22.1 | 22.0 | 21.8 | 21.1 | 20.4 | 13.8 | 22.5 |
| | Secretion efficiency (%) | 56 | 61 | 65 | 75 | 66 | 58 | 60 | 61 | 59 | 56 | 52 | 50 |

[Table 2]

| | | Example | | | | | | | | | | | Comparative Example |
|---|---|---|---|---|---|---|---|---|---|---|---|---|---|
| | | 12 | 13 | 14 | 15 | 16 | 17 | 18 | 19 | 20 | 21 | 22 | 2 |
| Surfactant | Surfactant (A1-1) | - | - | - | - | 0.03 | - | - | - | - | - | - | - |
| | Surfactant (A1-2) | - | - | - | - | - | 0.03 | - | - | - | - | - | - |
| | Surfactant (A2-1) | - | - | - | - | - | - | - | - | - | - | 0.03 | - |
| | Surfactant (A3-1) | - | - | - | - | - | - | 0.03 | - | - | - | - | - |
| | Surfactant (A3-2) | 0.003 | 0.03 | 0.3 | 3 | - | - | - | - | - | - | - | - |
| | Surfactant (A3-3) | - | - | - | - | - | - | - | 0.03 | - | - | - | - |
| | Surfactant (A3-4) | - | - | - | - | - | - | - | - | 0.03 | - | - | - |
| | Surfactant (A3-5) | - | - | - | - | - | - | - | - | - | 0.03 | - | - |
| Evaluation results | Optical density (OD) of broth at the end of culturing | 22.2 | 21.1 | 18.1 | 10.5 | 26.8 | 29.1 | 27.0 | 27.6 | 30.7 | 26.1 | 15.8 | 25 |
| | Secretion efficiency (%) | 64 | 80 | 84 | 67 | 66 | 64 | 62 | 75 | 66 | 90 | 88 | 50 |

[Table 3]

| | | Example | | | | | | | | | | | Comparative Example |
|---|---|---|---|---|---|---|---|---|---|---|---|---|---|
| | | 23 | 24 | 25 | 26 | 27 | 28 | 29 | 30 | 31 | 32 | 33 | 3 |
| Surfactant | Surfactant (A1-1) | - | - | - | - | 0.03 | - | - | - | - | - | - | - |
| | Surfactant (A1-2) | - | - | - | - | - | 0.03 | - | - | - | - | - | - |
| | Surfactant (A2-1) | - | - | - | - | - | - | - | - | - | - | 0.03 | - |
| | Surfactant (A3-1) | - | - | - | - | - | - | 0.03 | - | - | - | - | - |
| | Surfactant (A3-2) | 0.003 | 0.03 | 0.3 | 3 | - | - | - | - | - | - | - | - |
| | Surfactant (A3-3) | - | - | - | - | - | - | - | 0.03 | - | - | - | - |
| | Surfactant (A3-4) | - | - | - | - | - | - | - | - | 0.03 | - | - | - |
| | Surfactant (A3-5) | - | - | - | - | - | - | - | - | - | 0.03 | - | - |
| Evaluation results | Optical density (OD) of broth at the end of culturing | 5.1 | 6.0 | 2.7 | 2.7 | 5.77 | 5.68 | 5.7 | 8.07 | 7.91 | 7.33 | 3.94 | 5.01 |
| | Secretion efficiency (%) | 49 | 63 | 77 | 79 | 49 | 48 | 57 | 54 | 54 | 61 | 61 | 50 |

The surfactants described in Tables 1 to 3 are as follows.

Surfactant (A1-1): sodium polyoxyethylene (average 2.5 mol adduct) lauryl ether sulfate; pKa of the ionizable group in water at 25°C: 2.0

Surfactant (A1-2): sodium polyoxyethylene (average 3 mol adduct) lauryl ether acetate; pKa of the ionizable group

in water at 25°C: 3.6
Surfactant (A2-1): lauryl trimethyl ammonium chloride; pKa of the ionizable group in water at 25°C: 14
Surfactant (A3-1): sodium (2-dodecylamino)ethanesulfonate; pKa of the ionizable group in water at 25°C: 2.0 and 10.8
Surfactant (A3-2): sodium dodecyl-β-aminopropionate; pKa of the ionizable group in water at 25°C: 3.6 and 10.8
Surfactant (A3-3): dodecyldimethyl(3-sulfopropyl)ammonium hydroxide (lauryl sulfobetaine); pKa of the ionizable group in water at 25°C: 2.0 and 14
Surfactant (A3-4): miltefosine; pKa of the ionizable group in water at 25°C: 2.2 and 14
Surfactant (A3-5): betaine lauryldimethylaminoacetate; pKa of the ionizable group in water at 25°C: 3.6 and 14

The numerical values in Tables 1 to 3 each represent the amount (wt%) of the surfactant in the broth based on the weight of the broth.
The secretion efficiency of the useful substance in the broth obtained in each of Examples 1 to 33 and Comparative Examples 1 to 3 was evaluated as follows. Tables 1 to 3 show the results.

<Method of measuring optical density of broth>

The optical density of the broth containing the microorganisms collected by sampling was measured in a quartz cell (light path of 1 cm) using a spectrophotometer (UV-1700, Shimadzu Corporation).
The broth was centrifuged at 1500 rpm at 4°C for 5 minutes, and the supernatant was discarded. The precipitate was resuspended in a saline solution in an amount equal to the amount of the sample solution, and was diluted in the saline solution to obtain an appropriate absorbance (0.1 to 0.8). Then, the absorbance at 600 nm was measured. The optical density of the broth was calculated using the following mathematical formula (1).

Broth optical density (OD) = (Absorbance of diluted broth at 600 nm) × dilution factor of broth (Mathematical formula 1)

<Evaluation of secretion efficiency of useful substance>

Measurement of luciferase activity of secreted and produced useful substance

The broth obtained in each of Examples 1 to 22 and Comparative Examples 1 and 2 was 10 to 1000-fold diluted in a 0.2 M Tris-HCl buffer (pH 7.4), and 20 μl of the dilution was mixed with 60 μl of a luciferin solution described below to obtain a sample. The emission intensity of the sample was measured using a luminometer under the following conditions, and the measured emission intensity was substituted into the following formula. The resulting value was regarded as the luciferase activity of the secreted and produced useful substance.
Luminometer: "Glomax Navigator" available from Promega Corporation
Luciferin solution: product of New England Biolabs Japan Inc.
Measurement temperature: 25°C
Detector: emission detector
Detection wavelength: 350 to 700 nm

(Luciferase activity of secreted and produced useful substance) = (Emission intensity) × dilution factor/(optical density at the end of culturing)

Measurement of luciferase activity of useful substance remaining on bacterial surface

The bacteria obtained in each of Examples 1 to 22 and Comparative Examples 1 and 2 were mixed with a 0.2 M Tris-HCl buffer (pH 7.4) in an amount of 200 μl that is equal to the amount of the removed supernatant, followed by centrifugation, whereby the bacteria were washed. The washed bacteria were resuspended in a 0.2 M Tris-HCl buffer (pH 7.4) in an amount of 200 μl that is equal to the amount of the washed supernatant, and 10 to 1000-fold diluted in a 0.2 M Tris-HCl buffer (pH 7.4). The resulting dilution was used as a sample for measuring the luciferase activity on the bacterial surface.

Then, 20 μl of the sample for measuring the luciferase activity on the bacterial surface was mixed with 60 μl of the luciferin solution to obtain a sample. The emission intensity of the sample was measured under the same conditions as for "Measurement of luciferase activity of secreted and produced useful substance", and the measured emission intensity was substituted into the following formula. The resulting value was regarded as the luciferase activity of the useful substance remaining on the bacterial surface.

$$\text{(Luciferase activity of useful substance remaining on bacterial surface)} = \text{(Emission value)} \times \text{dilution factor}/\text{(optical density at the end of culturing)}$$

Secretion efficiency

Using the supernatant and the bacteria obtained in each of Examples 1 to 22 and Comparative Examples 1 and 2, the secretion efficiency in each example was calculated from the "luciferase activity of secreted and produced useful substance" and the "luciferase activity of useful substance remaining on bacterial surface" determined above, using the following formula. Tables 1 and 2 show the results.

$$\text{Secretion efficiency (\%)} = 100 \times \text{(Luciferase activity of secreted and produced useful substance)}/\text{(total luciferase activity)}$$

The total luciferase activity means the sum of the "luciferase activity of secreted and produced useful substance" and the "luciferase activity of useful substance remaining on bacterial surface".

<Measurement of amount of protein: measurement of acid phosphatase activity>

The supernatant obtained in each of Examples 23 to 33 and Comparative Example 3 was mixed with a 2 M sodium acetate buffer (pH 4.0) in an amount of 5 μl that is 1/40 of the amount of the supernatant. The buffer-containing supernatant or the sample for measuring the bacterial surface obtained in each of Examples 23 to 33 was mixed with a substrate solution (50 mM sodium acetate buffer containing 0.64 mg/ml of p-nitrophenylphosphate; pH 4.0) at a capacity ratio of 1:1, and the mixture was reacted at 35°C for 10 minutes.

After the reaction, a 10% solution of trichloroacetic acid in an amount equal to the amount of the reaction solution was added to the reaction solution to terminate the reaction. To develop a color, a saturated sodium carbonate solution in an amount equal to the total amount of the reaction solution was added to the solution in which the reaction has been terminated. The color-developed solution was centrifuged at 1500 × g for 5 minutes to remove the insoluble fraction, and the absorbance at 420 nm was measured using a microplate reader "PowerWave" (Biotek). The reference wavelength was set to 630 nm.

The acid phosphatase activity of each of the supernatant and the sample for measuring the bacterial surface was calculated from the following formula. The thus-obtained acid phosphatase activity of the supernatant was regarded as the "acid phosphatase activity of secreted and produced useful substance" and the acid phosphatase activity of the sample for measuring the bacterial surface was regarded as the "acid phosphatase activity of useful substance remaining on bacterial surface".

$$\text{Acid phosphatase activity} = ((\text{Abs 420 nm}) - (\text{Abs 630 nm}))/\text{(optical density at the end of culturing)}$$

<Evaluation of secretion efficiency>

For each of Examples 23 to 33 and Comparative Example 3, the obtained value of the "acid phosphatase activity of secreted and produced useful substance" and the obtained value of the "acid phosphatase activity of useful substance remaining on bacterial surface" were used to calculate the secretion efficiency from the following mathematical formula. Table 3 shows the results.

Secretion efficiency (%) = 100 × (Acid phosphatase activity of secreted and produced useful substance)/(total acid phosphatase activity)

The "total acid phosphatase activity" means the sum of the "acid phosphatase activity of secreted and produced useful substance" and the "acid phosphatase activity of useful substance remaining on bacterial surface".

It is clear from Tables 1 to 3 that the presence of the surfactant (A) in the broth increases the secretion efficiency, thus resulting in a higher secretion efficiency of the useful substance from microorganisms.

INDUSTRIAL APPLICABILITY

The method of producing a useful substance of the present invention is useful for production of biopharmaceuticals.

## Claims

**1.** A method of producing a useful substance, comprising:

secreting and producing a useful substance in broth by microorganisms contained in the broth,
wherein the microorganisms comprise microorganisms of at least one genus selected from the group consisting of *Ogataea, Saccharomyces, Kluyveromyces, Hansenula, Pichia, Yarrowia,* and *Candida,* and the broth contains at least one surfactant (A) selected from the group consisting of an anionic surfactant (A1), a cationic surfactant (A2), and an amphoteric surfactant (A3).

**2.** The method of producing a useful substance according to claim 1,
wherein the amount of the surfactant (A) in the broth is 0.0001 to 20 wt% based on the weight of the broth.

**3.** The method of producing a useful substance according to claim 1 or 2,
wherein the useful substance is a protein.

**INTERNATIONAL SEARCH REPORT**

International application No.
PCT/JP2018/007424

A. CLASSIFICATION OF SUBJECT MATTER
Int. Cl. C12P21/02(2006.01)i, C12P21/00(2006.01)i

According to International Patent Classification (IPC) or to both national classification and IPC

B. FIELDS SEARCHED

Minimum documentation searched (classification system followed by classification symbols)
Int. Cl. C12P21/02, C12P21/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN), WPIDS/WPIX (STN)

C. DOCUMENTS CONSIDERED TO BE RELEVANT

| Category* | Citation of document, with indication, where appropriate, of the relevant passages | Relevant to claim No. |
|---|---|---|
| X/Y | JP 61-043986 A (TAKARA SHUZO CO.) 03 March 1986, claims, page 3, lower right column to page 4, upper right column (Family: none) | 1-3/1-3 |
| Y | JP 2010-233513 A (SANYO CHEMICAL INDUSTRIES, LTD.) 21 October 2010, claims, examples (Family: none) | 1-3 |
| Y | JP 2015-091227 A (SANYO CHEMICAL INDUSTRIES, LTD.) 14 May 2015, claims, examples (Family: none) | 1-3 |
| Y | WO 2010/137624 A1 (SANYO CHEMICAL INDUSTRIES, LTD.) 02 December 2010, claims, examples & US 2012/0129220 A1, claims, examples & EP 2436773 A1 | 1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18.05.2018 | 29.05.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/007424

C (Continuation). DOCUMENTS CONSIDERED TO BE RELEVANT

| Category* | Citation of document, with indication, where appropriate, of the relevant passages | Relevant to claim No. |
|---|---|---|
| X/Y | DEMEZUK, A. et al., The effect of some conditions on the secretion of extracellular beta-galactosidase synthesised by Kluyveromyces fragilis 28, Communications in agricultural and applied biological sciences, 2003, vol. 68, pp. 469-472, ISSN 1379-1176, page 470, lines 34-38 | 1-3/1-3 |
| X/Y | SINGH, R. S. et al., Production of inulinase from Kluyveromyces marxianus YS-1 using root extract of Asparagus racemosus, Process Biochemistry, 2006, vol. 41, pp. 1703-1707, ISSN 1359-5113, fig. 2. | 1-3/1-3 |
| X/Y | ZEEV-BEN-MORDEHAI, T. et al., Amalgam, an axon guidance Drosophila adhesion protein belonging to the immunoglobulin superfamily= over-expression, purification and biophysical characterization, Protein Expr. Purif., 2009, vol. 63, pp. 147-157, ISSN 1046-5928, table 2 | 1-3/1-3 |
| X | WEI, G. et al., Effect of surfactants on extracellular accumulation of glutathione by Saccharomyces cerevisiae, Process Biochemistry, 2003, vol. 38, pp. 1133-1138, ISSN 1359-5113, fig. 4. | 1-3 |
| X | JP 48-056891 A (TANABE SEIYAKU CO., LTD.) 09 August 1973, claims, examples (Family: none) | 1-3 |
| X | JP 45-029436 B1 (KYOWA HAKKO KOGYO CO., LTD.) 25 September 1970, claims, examples (Family: none) | 1-3 |
| X | JP 50-154486 A (HITACHI CHEMICAL CO., LTD.) 12 December 1975, claims, examples (Family: none) | 1-3 |
| Y | AREVALO-VILLENA, M. et al., Pectinases yeast production using grape skin as carbon source, Advances in Bioscience and Biotechnology, 2011, vol. 2, pp. 89-96, ISSN 2156-8502, table 3. | 1-3 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *J. Bacteriol.,* 1998, vol. 180 (24), 6736-6742
- **H. YURIMOTO et al.** *Biochimica et Biophysica Acta,* 2000, vol. 1493, 56-63